# EUROPEAN PATENT APPLICATION

(11) **EP 4 501 344 A1**
(43) Date of publication of application: **05.02.2025**
(21) Application number: 23781240.9
(22) Date of filing: 23.03.2023
(51) Int. Cl.: A61K 38/10, A61P 11/00, A61P 29/00, A23L 33/18

(54) **PEPTIDE HAVING ANTI-INFLAMMATORY AND ANTI-FIBROTIC ACTIVITIES AND USE THEREOF**

(30) Priority: 31.03.2022 KR 20220040544
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); KIM, Seon Soo, Anyang-si, Gyeonggi-do 14119 (KR)
(74) Representative: Heinonen & Co
(86) International application number: PCT/KR2023/003862
(87) International publication number: WO 2023/191384

(57) **Abstract**

A peptide according to the present invention has the activity of inhibiting the expression of inflammatory cytokine genes, inflammatory cytokine proteins, or proteins involved in inflammatory cytokine signaling in lung cells in which inflammatory responses are induced. In addition, the peptide can reduce the number of neutrophils in the lung tissue in which inflammatory responses are induced. Therefore, the peptide according to the present invention can be used as an active substance useful for the treatment, prevention, or amelioration of lung inflammatory diseases.

## Description

### TECHNICAL FIELD

### Cross-reference to Related Applications

This application Claims the benefit of Korean Patent Application No. 10-2022-0040544, filed on March 31, 2022, in the Korean Intellectual Property Office, the disclosure of which is incorporated in the present specification in its entirety by reference.

### Technical Field

The present invention relates to a peptide having anti-inflammatory and anti-fibrotic activities and use thereof. More specifically, the present invention relates to a peptide having activity in treatment, prevention, or amelioration of lung inflammatory diseases or pulmonary fibrosis and use thereof.

### BACKGROUND ART

The human lung occupies 6% of the volume of the body and is composed of a lot of alveoli, which are small gas sacs. The primary intended purpose of the lung is to facilitate gas exchange with the systemic circulatory system. In a case where the lung is stimulated for any reason to cause an inflammation response, cytokines or growth factors are released from neutrophils, macrophages, neutrophilic cells, and mast cells, which are immune cells in the lung, and various lung cells. Inflammatory cytokines are known to be important mediator substances of inflammatory diseases. Cytokines secreted by inflammatory cells make lung epithelial cells produce and secrete interleukins, chemokines, colony stimulating factors, growth factors, and the like, and thus the lung epithelial cells play an important role in the network of these cytokines at local inflammation sites.

Asthma and chronic obstructive pulmonary disease (COPD), which are representative inflammatory diseases in the lung, are both diseases that are caused by chronic airway inflammation, and they are very common worldwide, and the prevalence thereof is rapidly increasing. In asthma patients, eosinophils, mast cells, and CD4+T cells invade the mucosa and submucosa layer of the airways, and these cells produce and secrete IL-4, IL-5, and IL-13. In addition, the inflammatory response in the lung is known to be a very important cause of pulmonary fibrosis. Most organs go through inflammation and curing processes after tissue damage. By the way, although normal structure and function are maintained in a case where the damage is minor, the tissue goes through fibrosis during the curing process in a case where there is continued damage.

As therapeutic agents for lung inflammatory diseases, which are currently used, corticosteroid formulations are most commonly used as anti-inflammatory agents for reducing inflammation. In addition, therapeutic agents serving as drugs targeting immune responses, which activate the Th1 response or inhibit the Th2 responses, and therapeutic agents such as anti-IL-2, anti-IL-4, anti-IL-5, anti-IL-9, anti-IL-13, anti-PGD2, and anti-TNF-α agents, which serve as cytokine inhibitors, a mast cell inhibitor, a PPAR-γ promoter, and anti-IgE and anti-CD23 agents are being developed. However, these drugs cause side effects in actual clinical practice or have weak actual therapeutic effects, and thus there is an urgent need to develop a new drug that is safer, exhibits higher efficacy, and is more economical in terms of cost.

### Documents of Related Art

[Patent Document 1] WO 2011/029931

### DISCLOSURE OF THE INVENTION

### TECHNICAL PROBLEM

As a result of research and efforts to develop a peptide having an anti-inflammatory activity, particularly an activity capable of inhibiting inflammation in the lung and inhibiting the progression of pulmonary fibrosis, the inventors of the present invention proved that, through lung cell and animal experiments, the peptide according to the present invention significantly alleviates the inflammatory response in the lung and also effectively inhibits the progression of pulmonary fibrosis, whereby the present invention has been completed.

As a result, an object of the present invention is to provide a pharmaceutical composition for treatment or prevention of lung inflammatory disease.

Another object of the present invention is to provide a functional food composition for amelioration or prevention of lung inflammation.

Still another object of the present invention is provide a functional food composition for amelioration or prevention of pulmonary fibrosis.

### TECHNICAL SOLUTION

In order to achieve the above objects, one aspect of the present invention provides a pharmaceutical composition for treatment or prevention of a lung inflammatory disease, and the pharmaceutical composition includes a peptide having an amino acid sequence of SEQ ID NO: 1 as an active ingredient.

Another aspect of the present invention provides a functional food composition for amelioration or prevention of lung inflammation, and the functional food composition includes a peptide having an amino acid sequence of SEQ ID NO: 1 as an active ingredient.

Still another aspect of the present invention provides a functional food composition for amelioration or prevention of pulmonary fibrosis, and the functional food composition includes a peptide having an amino acid sequence of SEQ ID NO: 1 as an active ingredient.

Hereinafter, the present invention will be described in detail.

### 1. Peptide and activity thereof

According to one aspect of the present invention, there is provided a peptide having an amino acid sequence set forth in SEQ ID NO: 1.

In the present specification, the term "peptide" means a linear molecule that is obtained by linking amino acid residues to each other through a peptide bond.

The peptide having the amino acid sequence of SEQ ID NO: 1 according to the present invention can be used without modification; however, a variant or fragment of amino acids having a different sequence due to deletion, insertion, or substitution of the amino acid residue, or a combination thereof can be used within a range in which the original activities of the peptide, for example, anti-diabetic and anti-obesity activities are not affected.

The peptide according to the present invention can be modified by phosphorylation, sulfation, acrylation, glycosylation, methylation, farnesylation, or the like, within a range in which the activity of the peptide is not changed.

The peptide according to the present invention includes a peptide having an amino acid sequence substantially identical to the peptide having the amino acid sequence of SEQ ID NO: 1, and a variant thereof, or an active fragment thereof. The substantially identical amino acid sequence means an amino acid sequence having a sequence identity of 75% or more, for example, 80% or more, 85% or more, 90% or more, 95% or more, or 97% or more, with the amino acid sequence of SEQ ID NO: 1. In addition, the peptide may additionally include a targeting sequence, a tag, a labeled residue, or an amino acid sequence produced for a characteristic purpose of increasing half-life or peptide stability.

The peptide according to the present invention may be such as one that is induced to have N-terminal and/or C-terminal modification in order to select a portion of the amino acid sequence and increase the activity thereof. Through this N-terminal and/or C-terminal modification, the stability of the peptide according to the present invention can be significantly improved, and for example, the half-life of the peptide can be increased in a case where the peptide is administered in vivo. The term "stability" is meant to include not only in vivo stability, which protects the peptide according to the present invention from attack by protein-cleaving enzymes in vivo, but also storage stability (for example, storage stability at room temperature)

The N-terminal modification may be such as one that is obtained by bonding a protecting group selected from the group consisting of an acetyl group, a fluoreonylmethoxycarbonyl group, a formyl group, a palmitoyl group, a myristyl group, a stearyl group, and polyethylene glycol (PEG), at the N-terminal of the peptide. The C-terminal modification may be such as one that is obtained by bonding a hydroxyl group (-OH), an amino group (-NH2), an azide (-NHNH2), or the like at the C-terminal of the peptide; however, the C-terminal modification is not limited thereto.

The peptide according to the present invention can be prepared by various methods that are widely known in the technical field to which the present invention belongs. For example, the peptide according to the present invention can be synthesized according to a chemical synthesis method publicly known in the art, particularly solid-phase synthesis techniques (Merrifield, J. Amer. Chem. Soc. 85: 2149-54 (1963); Stewart, et al., Solid Phase Peptide Synthesis, 2nd. ed., Pierce Chem. Co.: Rockford, 111(1984)) or a liquid synthesis technique (US Patent No. 5,516,891).

The peptide according to the present invention has the activity of inhibiting the expression of inflammatory cytokine genes, inflammatory cytokine proteins, or proteins involved in inflammatory cytokine signaling in lung cells in which inflammatory responses are induced.

In one embodiment, the inflammatory cytokine gene of which the expression is inhibited may be one or more selected from the group consisting of TNFα, IL-1β, IL-6, IL-8, and COX-2 genes.

In one embodiment, the inflammatory cytokine protein of which the expression is inhibited, or the protein involved in the inflammatory cytokine signaling may be one or more selected from the group consisting of IL-1β, IL-6, COX2, IL-8, p-JNK, p-ERK, p-PI3K, p-AKT, p-ERK, and myeloperoxidase.

The peptide according to the present invention inhibits the expression of a fibrosis gene in lung cells in which an inflammatory response is induced.

In one embodiment, the fibrosis gene of which the expression is inhibited may be one or more selected from the group consisting of α-SMA, Collagen 1A1, Fibronectin 1, and Tenascin C genes.

The peptide according to the present invention can reduce the number of neutrophils in a lung tissue in which inflammatory responses are induced.

Due to having such activity as described above, the above-described peptide according to the present invention can exhibit excellent efficacy in treating, preventing, or ameliorating lung inflammatory diseases.

### 2. Composition for treatment or prevention of lung inflammatory disease

### Pharmaceutical composition

According to another aspect of the present invention, there is provided a pharmaceutical composition for treatment or prevention of a lung inflammatory disease, the pharmaceutical composition comprising a peptide having an amino acid sequence of SEQ ID NO: 1 as an active ingredient.

As described above, since the peptide according to the present invention inhibits, in lung cells in which inflammatory responses are induced, the expressions of inflammatory cytokine genes, inflammatory cytokine proteins, or proteins involved in inflammatory cytokine signaling, it has excellent activity in treatment or prevention of lung inflammatory diseases.

In one embodiment of the present invention, the lung inflammatory disease may be a disease selected from the group consisting of asthma, allergic asthma, bronchitis, chronic bronchitis, acute bronchitis, bronchiolitis, diffuse panbronchiolitis, bronchiectasis, pleurisy, alveolitis, vasculitis, pneumonia, hypersensitivity pneumonia, chronic obstructive pulmonary disease, pulmonary fibrosis, idiopathic pulmonary fibrosis, cystic fibrosis, acute respiratory distress syndrome, acute lung injury, lung injury due to smoke inhalation, and heat-induced lung injury; however, the present invention is not limited thereto.

In the pharmaceutical composition for treatment or prevention of a lung inflammatory disease according to the present invention, the peptide can inhibit the expression of inflammatory cytokine genes, inflammatory cytokine proteins, or proteins involved in inflammatory cytokine signaling in lung cells in which inflammatory responses are induced.

The inflammatory cytokine gene may be one or more selected from the group consisting of TNFα, IL-1β, IL-6, IL-8, and COX-2 genes.

The inflammatory cytokine protein or the protein involved in the inflammatory cytokine signaling may be one or more selected from the group consisting of IL-1β, IL-6, COX2, IL-8, p-JNK, p-ERK, p-PI3K, p-AKT, p-ERK, and myeloperoxidase.

In the pharmaceutical composition for treatment or prevention of a lung inflammatory disease according to the present invention, the peptide can inhibit an expression of a fibrosis gene in lung cells in which an inflammatory response is induced.

The fibrosis gene may be one or more selected from the group consisting of α-SMA, Collagen 1A1, Fibronectin 1, and Tenascin C genes.

In the pharmaceutical composition for treatment or prevention of a lung inflammatory disease according to the present invention, the peptide can reduce the number of neutrophils in the lung tissue in which inflammatory responses are induced.

The pharmaceutical composition according to the present invention may be such as one that contains a therapeutically effective amount of the above-described peptide and a pharmaceutically acceptable carrier

The term "therapeutically effective amount" means an amount that makes a peptide sufficiently achieve the activity or efficacy thereof, where the peptide is an active ingredient of the pharmaceutical composition according to the present invention. For example, it means an amount that is sufficient for achieving the efficacy of treatment or prevention of lung inflammatory diseases.

The pharmaceutically acceptable carrier is such as one that is commonly used in formulation, and it includes lactose, dextrose, sucrose, sorbitol, mannitol, starch, Acacia rubber, calcium phosphate, alginate, gelatin, calcium silicate, microcrystalline cellulose, polyvinyl pyrrolidone, cellulose, water, syrup, methyl cellulose, methylhydroxybenzoate, propylhydroxybenzoate, talc, magnesium stearate, mineral oil, and the like; however, the pharmaceutically acceptable carrier is not limited thereto.

In addition to the above ingredients, the pharmaceutical composition according to the present invention may further include, but not limited to, a lubricant, a wetting agent, a sweeting agent, a flavoring agent, an emulsifier, a suspending agent, a preservative, and the like.

The suitable pharmaceutically acceptable carriers and formulations are described in detail in Remington: The Science and Practice of Pharmacy, (19th ed., 1995, Williams & Wilkins).

The pharmaceutical composition according to the present invention can be administered via any route suitable for treating lung inflammatory diseases. For example, it can be administered orally or parenterally, and in a case of parenteral administration, it can be administered by intravenous injection, subcutaneous injection, intramuscular injection, intraperitoneal injection, topical administration, or transdermal administration.

The administration dosage of the pharmaceutical composition may be 0.0001 µg to 100 mg, 0.001 µg to 100 mg, 0.01 µg to 100 mg, 0.1 µg to 100 mg, or 1.0 µg to 1000 mg per day; however, the administration dosage thereof is not limited thereto. The prescription can be made in various ways depending on factors such as formulation method, administration method, patient's age, body weight, sex, pathological condition, food, administration time, administration route, excretion rate, and reaction sensitivity.

The pharmaceutical composition according to the present invention may be produced in a unit dosage form or produced to be incorporated into a multi-dosage container by formulating it using a pharmaceutically acceptable carrier and/or excipient according to a method that can be easily carried out by those skilled in the art. In this case, the formulation form may be a form of a solution, suspension, or emulsion in an oily or aqueous medium or may be a form of an extract, a powder, a granule, a tablet, or a capsule, and a dispersant or stabilizer may be further contained.

### Food composition

According to another aspect of the present invention, there is provided a functional food composition for amelioration or prevention of lung inflammation, the functional food composition comprising a peptide having an amino acid sequence of SEQ ID NO: 1 as an active ingredient.

The lung inflammation may be pulmonary fibrosis due to a disease selected from the group consisting of asthma, allergic asthma, bronchitis, chronic bronchitis, acute bronchitis, bronchiolitis, diffuse panbronchiolitis, bronchiectasis, pleurisy, alveolitis, vasculitis, pneumonia, hypersensitivity pneumonia, chronic obstructive pulmonary disease, pulmonary fibrosis, idiopathic pulmonary fibrosis, cystic fibrosis, acute respiratory distress syndrome, acute lung injury, lung injury due to smoke inhalation, and heat-induced lung injury; however, the lung inflammation is not limited thereto.

According to still another aspect of the present invention, there is provided a functional food composition for amelioration or prevention of pulmonary fibrosis, the functional food composition comprising a peptide having an amino acid sequence of SEQ ID NO: 1 as an active ingredient.

In the functional food composition for amelioration or prevention of lung inflammation or pulmonary fibrosis according to the present invention, the peptide can inhibit the expression of inflammatory cytokine genes, inflammatory cytokine proteins, or proteins involved in inflammatory cytokine signaling in lung cells in which inflammatory responses are induced.

The inflammatory cytokine gene may be one or more selected from the group consisting of TNFα, IL-1β, IL-6, IL-8, and COX-2 genes.

The inflammatory cytokine protein or the protein involved in the inflammatory cytokine signaling may be one or more selected from the group consisting of IL-1β, IL-6, COX2, IL-8, p-JNK, p-ERK, p-PI3K, p-AKT, p-ERK, and myeloperoxidase.

In the functional food composition for amelioration or prevention of lung inflammation or pulmonary fibrosis according to the present invention, the peptide can inhibit an expression of a fibrosis gene in lung cells in which an inflammatory response is induced.

The fibrosis gene may be one or more selected from the group consisting of α-SMA, Collagen 1A1, Fibronectin 1, and Tenascin C genes.

In the functional food composition for amelioration or prevention of lung inflammation or pulmonary fibrosis according to the present invention, the peptide can reduce the number of neutrophils in the lung tissue in which inflammatory responses are induced.

In the functional food composition according to the present invention, an appropriate amount of the peptide may be selected and contained within a range of 0.0001% by weight to 10% by weight with respect to the total weight of the composition; however, the amount of the peptide is not limited thereto.

In one embodiment, the functional food composition according to the present invention may be such as one that contains a sitologically effective amount of the above-described peptide and a sitologically acceptable carrier.

The food composition according to the present invention contains not only a peptide as the active ingredient but also ingredients that are commonly added during food production, and it may contain, for example, a protein, a carbohydrate, a fat, a nutrient, a seasoning, and a flavoring agent. Examples of the above-described carbohydrate include monosaccharides, for example, glucose and fructose; disaccharides, for example, maltose, sucrose, and oligosaccharide; and polysaccharides, for example, conventional sugars such as dextrin and cyclodextrin, and sugar alcohols such as xylitol, sorbitol, and erythritol. As the flavoring agent, a natural flavoring agent, thaumatin, a stevia extract (for example, rebaudioside A or glycyrrhizin) and a synthetic flavoring agent (saccharin, aspartame, or the like) can be used. The ratio of the carbohydrate may generally be about 1 to 20 g and desirably about 5 to 12 g per 100 g of the food composition according to the present invention; however, the ratio thereof is not limited thereto.

In addition to the above-described ingredients, the functional food composition according to the present invention may contain various nutritional supplements, a vitamin, a mineral (electrolyte), flavoring agents such as a synthetic flavoring agent and a natural flavoring agent, a colorant and a thickening agent (cheese, chocolate, or the like), pectic acid and a salt thereof, alginic acid and a salt thereof, an organic acid, a protective colloidal thickener, a pH adjusting agent, a stabilizer, a preservative, glycerin, alcohol, a carbonating agent used in a carbonated drink, and the like. In addition, it may contain pulp for the production of a natural fruit juice, a fruit juice drink, and a vegetable drink. For example, in a case where the functional food composition according to the present invention is produced as a drink, it may contain citric acid, high-fructose corn syrup, sugar, glucose, acetic acid, malic acid, a fruit juice, an Eucommia extract, a jujube extract, a licorice extract, and the like, in addition to the peptide, which is the active ingredient according to the present invention.

Another aspect of the present invention provides a method for treatment, prevention, or amelioration of lung inflammatory diseases, which includes a step of administering, into a patient with a lung inflammatory disease, a therapeutically effective amount of the above-described peptide having the amino acid sequence of SEQ ID NO: 1 or a therapeutically effective amount of a pharmaceutical composition containing the above-described peptide.

A still another aspect of the present invention provides the use of the above-described peptide having the amino acid sequence of SEQ ID NO: 1, where the use is for treating, preventing, or ameliorating lung inflammatory diseases.

A still another aspect of the present invention provides the use of a medicament for treating, preventing, or ameliorating lung inflammatory diseases, or the use of the above-described peptide having the amino acid sequence of SEQ ID NO: 1, which is for producing a functional food composition.

To the above-described method for treatment, prevention, or amelioration of lung inflammatory diseases, the contents described above regarding the peptide and pharmaceutical composition according to the present invention can be equally applied and are not described repeatedly to avoid excessive complexity of the specification.

### ADVANTAGEOUS EFFECTS

A peptide according to the present invention has the activity of inhibiting the expression of inflammatory cytokine genes, inflammatory cytokine proteins, or proteins involved in inflammatory cytokine signaling in lung cells in which inflammatory responses are induced. In addition, the peptide can reduce the number of neutrophils in the lung tissue in which inflammatory responses are induced. Therefore, the peptide according to the present invention can be used as an active substance useful for the treatment, prevention, or amelioration of lung inflammatory diseases.

However, the effect of the present invention is not limited to the effects mentioned above, and other effects that are not mentioned will be clearly understood by those skilled in the art from the following description.

### BRIEF DESCRIPTION OF THE DRAWINGS

FIG. 1 shows the results obtained by measuring the effect of treatment with a peptide of Production Example 1 on gene expression of inflammatory cytokines TNF-α, IL-1β, IL-6, and IL-8 in MRC-5 cells in which an inflammatory response has been induced by an LPS treatment.
FIG. 2 shows the results obtained by measuring the effect of treatment with the peptide of Production Example 1 on the gene expression of inflammatory cytokines TNF-α, IL-1β, IL-6, IL-8, and COX-2 in A549 cells in which an inflammatory response has been induced by an LPS treatment.
FIG. 3 shows the results obtained by measuring the effect of treatment with the peptide of Production Example 1 on the expression of IL-1β, IL-6, pJNK, and pERK in MRC-5 cells in which an inflammatory response has been induced by an LPS treatment.
FIG. 4a and FIG. 4b show the results obtained by measuring the effect of treatment with the peptide of Production Example 1 on the expression of Phospho-PI3K, Phospho-AKT, Phospho-ERK, IL-1β, IL-6, and COX2 in A549 cells in which an inflammatory response has been induced by an LPS treatment.
FIG. 5a and FIG. 5b show the results obtained by measuring the effect of treatment with the peptide of Production Example 1 on the expression of IL-6 and IL-8 in MRC-5 cells in which an inflammatory response has been induced by an LPS treatment.
FIG. 6 shows the results obtained by measuring the effect of treatment with the peptide of Production Example 1 on the expression levels of the α-SMA and Collagen 1A1 gene as fibrosis genes in MRC-5 cells in which an inflammatory response has been induced by an LPS treatment.
FIG. 7 shows the results obtained by measuring the effect of treatment with the peptide of Production Example 1 on the expression levels of the α-SMA, Fibronectin 1, Collagen 1A1, and Tenascin C genes as fibrosis genes in MRC-5 cells treated with TGF-β1.
FIG. 8 shows the results obtained by measuring the effect of treatment with the peptide of Production Example 1 on the gene expression levels of inflammatory cytokines TNF-α, IL-1β, IL-6, IL-8, and COX-2 in A549 cells treated with IL-1β.
FIG. 9a, FIG. 9b, and FIG. 9c show the results obtained by measuring the effect of treatment with the peptide of Production Example 1 on the expression levels of inflammatory cytokines IL-1β, IL-6, and IL-8 in A549 cells treated with IL-1β.
FIG. 10a and FIG. 10b show the results obtained by measuring the effect of treatment with the peptide of Production Example 1 on the total number of cells, the number of neutrophils, and the number of macrophages in the bronchoalveolar lavage fluid of mice in which lung inflammation has been induced by LPS.
FIG. 11a to FIG. 11d show the results obtained by measuring the effect of treatment with the peptide of Production Example 1 on the expression levels of TNF-α, IL-1β, IL-6, and Myeloperoxidase (MPO) in the bronchoalveolar lavage fluid of mice in which lung inflammation has been induced by LPS.
FIG. 12a and FIG. 12b show the results obtained by observing H&E staining in the lung tissue of mice in which lung inflammation response has been induced by LPS, the results obtained by measuring the thickness of the alveolar wall of the mice, the results obtained by measuring the total number of cells of the mice, and the results obtained by measuring the number of alveoli of the mice.
FIG. 13a shows the results obtained by measuring the body weight of mice in which pulmonary fibrosis has been induced by BLM (Bleomycin), FIG. 13b shows the results obtained by measuring the effect of treatment with the peptide of Production Example 1 on the number of macrophages and lymphocytes in the bronchoalveolar lavage fluid of the mice, and FIG. 13c and FIG. 13d show the results obtained by observing cells with a microscope and the results obtained by measuring the total number of cells in the bronchoalveolar lavage fluid of the mice.
FIG. 14a shows the results obtained by observing H&E staining in the lung tissue of each treatment group in which pulmonary fibrosis has been induced by BLM (Bleomycin), and FIG. 14b shows the results obtained by measuring the number of alveoli in each treatment group in an image of the lung tissue that has been subjected to the H&E-staining, the results obtained by measuring the total number of cells in each treatment group, and the results obtained by measuring the thickness of the alveolar wall in each treatment group.
FIG. 15a shows the results obtained by observing, after carrying out Trichrome staining, the lung tissue of mice of each treatment group in which pulmonary fibrosis has been induced by BLM (Bleomycin), and FIG. 15b shows the change (in terms of %) in the amount of collagen in the remaining treatment groups comparative to the normal group, where the amount of collagen in the normal group is set to 100%.

### MODE FOR CARRYING OUT THE INVENTION

Hereinafter, the present invention will be described in detail according to examples. However, the following examples specifically illustrate the present invention, and thus the contents of the present invention are not limited by the following examples.

### Production Example 1: Production of peptide

A peptide having the amino acid sequence of SEQ ID NO: 1 described in Table 1 below was synthesized using an automatic peptide synthesizer (Milligen 9050, Millipore, USA), and the synthesized peptide was purified using C18 reversed-phase high-performance liquid chromatography (HPLC) (Waters Associates, USA). The column used was ACQUITY UPLC BEH300 C18 (2.1 mm X 100 mm, 1.7 µm, Waters Co, USA).

**[Table 1]**

| SEQ ID NO | Amino acid sequence of peptide |
|---|---|
| 1 | KYLLVHRPYYRR |

Next, the efficacy of the prepared peptide of SEQ ID NO: 1 was evaluated.

### Experimental Example 1: Inhibition of inflammatory cytokine gene expression induced by LPS

It was tested whether the peptide of SEQ ID NO: 1 prepared in Production Example 1 inhibits the expression of inflammatory cytokine genes in lung cells.

### 1. MRC-5 cell

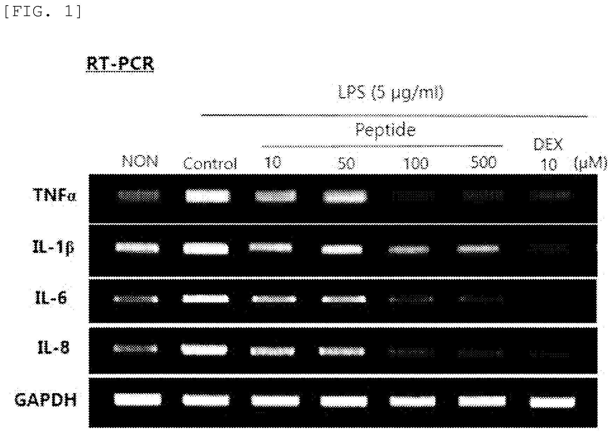

MRC-5 cells (lung fibroblast cells) were seeded at 3 × 10⁵ cells/well in a 12-well plate and used in the experiment. After culturing for 1 day with a culture medium containing 10% FBS, the culture medium was replaced with a culture medium without containing FBS to induce starvation for 4 hours. A normal group without being treated with any substances, a group treated with 5µg/ml of LPS (Sigma Aldrich) (negative control group), groups treated with 5µg/ml of LPS and the peptide of Production Example 1 (10, 50, 100, 500µM), and a group treated with 5µg/ml of LPS and 10 µM of Dexamethasone (DEX) (positive control group) were treated with active substances and drugs respectively, and then RNA was extracted after 24 hours to carry out RT-PCR. RNA was extracted from MRC-5 cells using an easy-BLUE^{™} Total RNA extraction kit (Qiagen, Germany). The extracted RNA was converted into cDNA with RT-PCR premix (iNtRON Biotechnology, Seongnam, Korea). After preparing a reaction mixture with PCR premix (iNtRON Biotechnology, Seongnam, Korea) and primers for each gene, PCR was carried out using a PCR machine (Eppendorf, Germany). Next, the mRNA expression pattern was determined by agarose gel electrophoresis. The base sequences of the primers used in the experiment are listed in [Table 2] below. As a result of the experiment, as shown in FIG. 1, it was confirmed that although the treatment with LPS, which is an inflammation-inducing factor, has increased the gene expression of inflammatory cytokines TNF-α, IL-1β, IL-6, and IL-8, the increased expression of the genes decreases in a concentration-dependent manner in a case where treatment is carried out with the peptide 1 of Production Example 1 is treated.

### 2. A549 cell

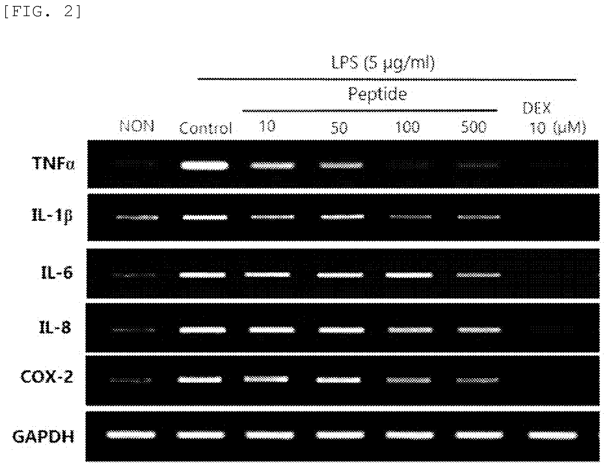

A549 cells (human alveolar basal epithelial cells) were seeded at 3 × 10⁵ cells/well in a 6-well plate and used in the experiment. After culturing for 1 day with a culture medium containing 10% FBS, the culture medium was replaced with a culture medium without containing FBS to induce starvation for 4 hours. A normal group without being treated with any substances, a group treated with 5µg/ml of LPS (Sigma Aldrich) (negative control group), groups treated with 5µg/ml of LPS and the peptide of Production Example 1 (10, 50, 100, 500µM), and a group treated with 5µg/ml of LPS and 10 µM of Dexamethasone (DEX) (positive control group) were treated with active substances and drugs respectively, and then RNA was extracted after 3 hours to carry out RT-PCR. RNA was extracted from the A549 cells in the above-described treatment group using an easy-BLUE^{™} Total RNA extraction kit (Qiagen, Germany). The extracted RNA was converted into cDNA with RT-PCR premix (iNtRON Biotechnology, Seongnam, Korea). After preparing a reaction mixture with PCR premix (iNtRON Biotechnology, Seongnam, Korea) and primers for each gene, PCR was carried out using a PCR machine (Eppendorf, Germany). Next, the mRNA expression pattern was determined by agarose gel electrophoresis. The base sequences of the primers used in the experiment are listed in [Table 2] below. As a result of the experiment, as shown in FIG. 2, it was confirmed that although the treatment with LPS, which is an inflammation-inducing factor, has increased the gene expression of inflammatory cytokines TNF-α, IL-1β, IL-6, IL-8, and COX-2, the increased expression of the genes decreases in a concentration-dependent manner in a case where treatment is carried out with the peptide 1 of Production Example 1 is treated.

**[Table 2]**

| Gene | Primer | Sequence (5' -> 3') | SEQ ID NO |
|---|---|---|---|
| TNF-α | F | AACATCCAACCTTCCCAAACG | 2 |
| | R | GACCCTAAGCCCCCAATTCTC | 3 |
| IL-1β | F | TTCGACACATGGGATAACGA | 4 |
| | R | TCTTTCAACACGCAGGACAG | 5 |
| IL-6 | F | AAA GAG GCA CTG CCA GAA AA | 6 |
| | R | ATC TGA GGT GCC CAT GCT AC | 7 |
| IL-8 | F | GAAGGTGCAGTTTTGCCAAG | 8 |
| | R | ACCCTCTGCACCCAGTTTTC | 9 |
| COX-2 | F | ATCATTCACCAGGCAAATTGC | 10 |
| | R | GGCTTCAGCATAAAGCGTTTG | 11 |
| GAPDH | F | GTG ATG GCA TGG ACT GTG GT | 12 |
| | R | GGA GCC AAA AGG GTC ATC AT | 13 |

### Experimental Example 2: Inhibition of inflammatory cytokine protein expression induced by LPS

It was tested whether the peptide of SEQ ID NO: 1 prepared in Production Example 1 inhibits the expression of inflammatory cytokine proteins in lung cells.

### 1. Western blotting analysis 1: MRC-5 cell

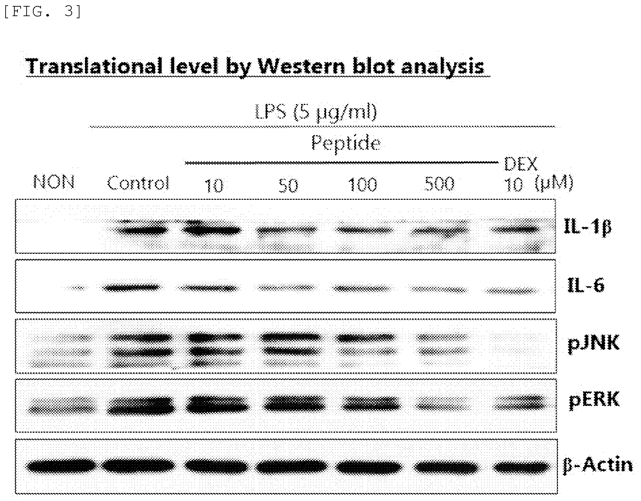

MRC-5 cells (lung fibroblast cells) were seeded at 2 × 10⁵ cells/well in a 6-well plate and used in the experiment. After culturing for 1 day with a culture medium containing 10% FBS, the culture medium was replaced with a culture medium without containing FBS to induce starvation for 4 hours. Cells of a normal group without being treated with any substances, a group treated with 5µg/ml of LPS (Sigma Aldrich) (negative control group), groups treated with 5µg/ml of LPS and the peptide of Production Example 1 (10, 50, 100, 500µM), and a group treated with 5µg/ml of LPS and 10 µM of Dexamethasone (DEX) (positive control group) were treated with active substances and drugs respectively and then cultured for 24 hours. The cultured cells of each treatment group and control group were lysed by adding a lysis buffer, and then centrifugation was carried out at 4°C at 12,000 rpm for 30 minutes to obtain proteins, which were subsequently quantified using a BCA Protein Assay kit (Sigma). The proteins were subjected to SDS-PAGE (sodium dodecyl sulfate-polyacrylamide gel electrophoresis) and then subjected to electrotransfer to a membrane. The membrane to which the proteins were attached was treated with 5% skim milk for blocking and then subjected to a reaction with a primary antibody overnight at 4°C. After washing with PBS-T, the membrane was subjected to a reaction with a secondary antibody at room temperature for 1 hour, washed again with PBS-T, and then visualized by a Western detection reagent (Elpis Biotech, Daejeon, Korea) using Gel Doc (Bio-Rad, Hercules, CA, USA). The antibodies used in the experiment are as follows. An Anti-IL-1β antibody (Cell signaling technology (CST), USA), an anti-IL-6 antibody (Santa Cruz Biotechnology, USA), an anti-pJNK antibody (Santa Cruz Biotechnology, USA), an anti-pERK antibody (Cell signaling technology (CST), USA), and an anti-β-Actin antibody (Santa Cruz Biotechnology, USA). As a result of the experiment, as shown in FIG. 3, although the expression of IL-1β, IL-6, pJNK, and pERK was increased in a case where treatment was carried out with LPS, the treatment with the peptide of Production Example 1 decreased again the expression of IL-1β, IL-6, pJNK, and pERK in a concentration-dependent manner.

### 2. Western blotting analysis 2: A549 cell

A549 cells (human alveolar basal epithelial cells) were seeded at 3 × 10⁵ cells/well in a 6-well plate and used in the experiment. After culturing for 1 day with a culture medium containing 10% FBS, the culture medium was replaced with a culture medium without containing FBS to induce starvation for 4 hours. Cells of a normal group without being treated with any substances, a group treated with LPS (5µg/ml) (Sigma Aldrich) (negative control group), groups treated with LPS (5µg/ml) and the peptide of Production Example 1 (10, 50, 100, 500µM), and a group treated with LPS (5µg/ml) and 10 µM of Dexamethasone (DEX) (positive control group) were treated with active substances and drugs respectively and then cultured for 24 hours. The cultured cells of each treatment group and control group were lysed by adding a lysis buffer, and then centrifugation was carried out at 4°C at 12,000 rpm for 30 minutes to obtain proteins, which were subsequently quantified using a BCA Protein Assay kit (Sigma). The proteins were subjected to SDS-PAGE (sodium dodecyl sulfate-polyacrylamide gel electrophoresis) and then subjected to electrotransfer to a membrane. The membrane to which the proteins were attached was treated with 5% skim milk for blocking and then subjected to a reaction with a primary antibody overnight at 4°C. After washing with PBS-T, the membrane was subjected to a reaction with a secondary antibody at room temperature for 1 hour, washed again with PBS-T, and then visualized by a Western detection reagent (Elpis Biotech, Daejeon, Korea) using Gel

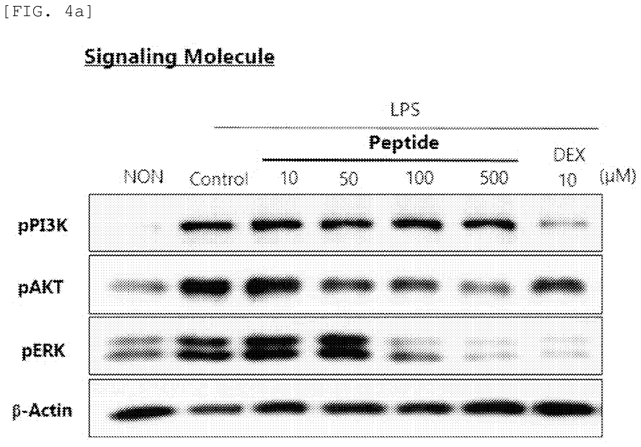

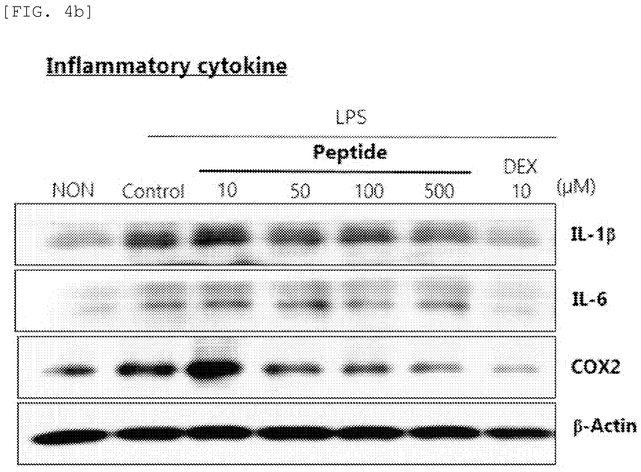

Doc (Bio-Rad, Hercules, CA, USA). The antibodies used in the experiment are as follows. An Anti-IL-1β antibody: (Cell signaling technology (CST), USA), an anti-IL-6 antibody: (Santa Cruz Biotechnology, USA), an anti-COX2 antibody: (Santa Cruz Biotechnology, USA), an anti-Phospho-PI3K antibody: (Cell signaling technology (CST), USA), an anti-Phospho-AKT antibody: (Cell signaling technology (CST), USA), an anti-Phospho-ERK antibody: (Cell signaling technology (CST), USA), and an anti-β-Actin antibody: (Santa Cruz Biotechnology, USA). As a result of the experiment, as shown in FIG. 4a and FIG. 4b, although the expression of Phospho-PI3K, Phospho-AKT, Phospho-ERK, IL-1β, IL-6, and COX2 was increased in a case where treated with LPS, the treatment with the peptide of Production Example 1 decreased the expression levels of proteins in a concentration-dependent manner.

### 3. ELISA analysis

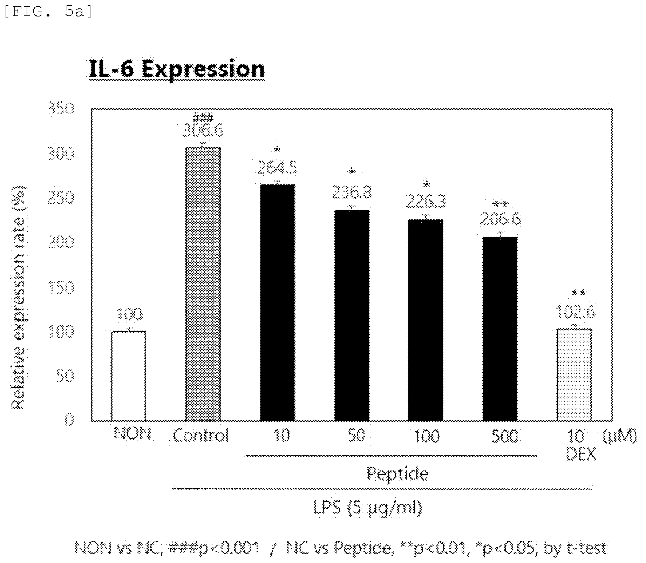

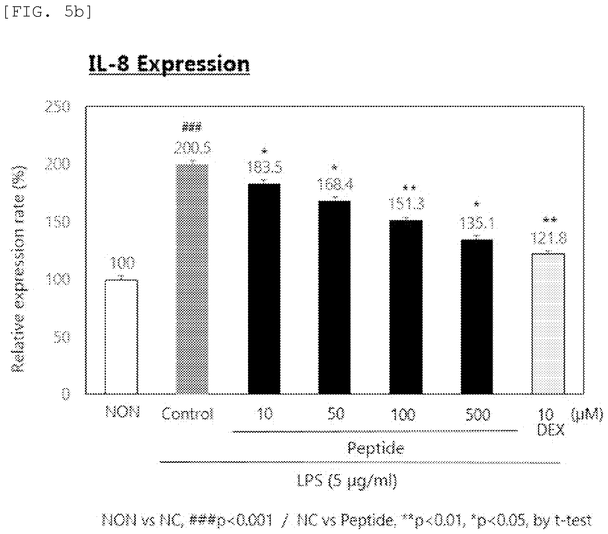

MRC-5 cells (lung fibroblast cells) were seeded at 2 × 10⁵ cells/well in a 12-well plate and used in the experiment. After culturing for 1 day with a culture medium containing 10% FBS, the culture medium was replaced with a culture medium without containing FBS to induce starvation for 4 hours. Cells of a normal group without being treated with any substances, a group treated with 5µg/ml of LPS (Sigma Aldrich) (negative control group), groups treated with 5µg/ml of LPS and the peptide of Production Example 1 (10, 50, 100, 500µM), and a group treated with 5µg/ml of LPS and 10µ M of Dexamethasone (DEX) (positive control group) were treated with active substances and drugs respectively and then cultured for 72 hours. After cell culture, the supernatant obtained by centrifuging the culture solution at 4°C and 3,000 rpm for 10 minutes was subjected to ELISA analysis using an IL-6 Quantikine ELISA Kit (R&D systems) and an IL-8 Quantikine ELISA kit (R&D systems) according to the method of use in the kit instructions, and then the absorbance was measured at 450 nm using a microplate reader. As a result of the experiment, as shown in FIG. 5a and FIG. 5b, although the expression levels of IL-6 and IL-8 were increased in a case where treatment was carried out with LPS, the treatment with the peptide of Production Example 1 decreased the expression levels of IL-6 and IL-8 in a concentration-dependent manner.

### Experimental Example 3: Inhibition of fibrosis gene expression induced by LPS

It was tested whether the peptide of SEQ ID NO: 1 produced in Production Example 1 inhibits the expression of fibrosis genes induced by LPS in lung cells. MRC-5 cells (lung fibroblast cells) were seeded at 2 × 10⁵ cells/well in a 12-well plate and used in the experiment. After culturing for 1 day with a culture medium containing 10% FBS, the culture medium was replaced with a culture medium without containing FBS to induce starvation for 4 hours. Cells of a normal group without being treated with any substances, a group treated with 5µg/ml of LPS (Sigma Aldrich) (negative control group), groups treated with 5µg/ml of LPS and the peptide of Production Example 1 (10, 50, 100, 500µM), and a group treated with 5µg/ml of LPS and 10 µM of Dexamethasone (DEX) (positive control group) were treated with the above-described active substances and drugs respectively, and then RNA was extracted after 3 hours to carry out RT-PCR. RNA was extracted from MRC-5 cells using an easy-BLUE^{™} Total RNA extraction kit (Qiagen, Germany). The extracted RNA was converted into cDNA using RT-PCR premix (iNtRON Biotechnology, Seongnam, Korea). After preparing a reaction mixture with PCR premix (iNtRON Biotechnology, Seongnam, Korea) and primers for each gene, PCR was carried out using a PCR machine (Eppendorf, Germany). Next, the mRNA expression pattern was determined by agarose gel electrophoresis. The base sequences of the primers used in the experiment are listed in Table 3 below.

**[Table 3]**

| Gene | Primer | Sequence (5' -> 3') | SEQ ID NO |
|---|---|---|---|
| α-SMA | F | GGTGCTGTCTCTCTATGCCTCTGGA | 14 |
| | R | CCCATCAGGCAACTCGTAACTCTTC | 15 |
| Collagen 1A1 | F | CATCACCTACCACTGCAAGAAC | 16 |
| | R | ACGTCGAAGCCGAATTCC | 17 |
| GAPDH | F | GTG ATG GCA TGG ACT GTG GT | 12 |
| | R | GGA GCC AAA AGG GTC ATC AT | 13 |

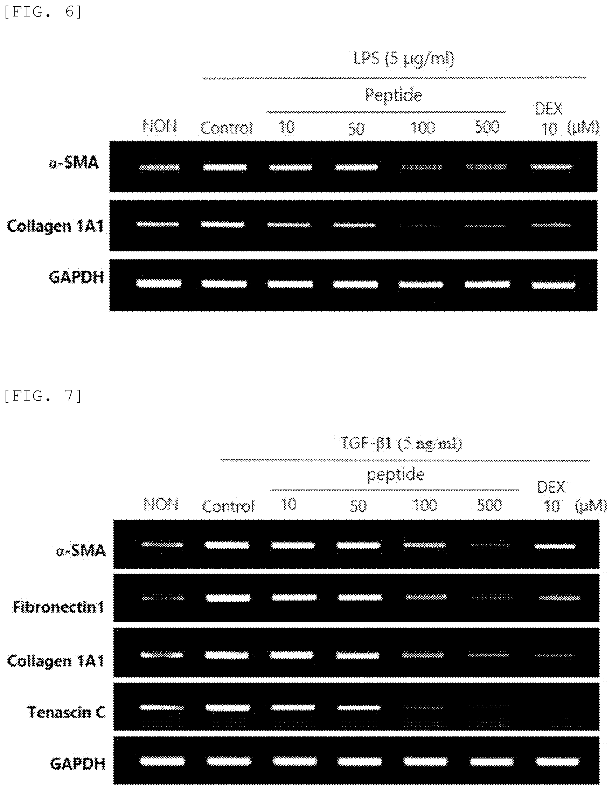

As a result of the experiment, as shown in FIG. 6, it was confirmed that although the expression levels of α-SMA and Collagen 1A1 genes have been increased in a case where treatment was carried out with LPS, the treatment with the peptide of Production Example 1 decreases again the expression levels of the α-SMA and Collagen 1A1 genes in a concentration-dependent manner.

### Experimental Example 4: Inhibition of fibrosis gene expression induced by TGF-β1

It was tested whether the peptide of SEQ ID NO: 1 prepared in Production Example 1 inhibits the expression of fibrosis genes induced by TGF-β1 in lung cells. MRC-5 cells (lung fibroblast cells) were seeded at 2 × 10⁵ cells/well in a 12-well plate and used in the experiment. After culturing for 1 day with a culture medium containing 10% FBS, the culture medium was replaced with a culture medium without containing FBS to induce starvation for 4 hours. Cells of a normal group without being treated with any substances, a group treated with 5 ng/ml of TGF-β1 (negative control group), groups treated with 5 ng/ml of TGF-β1 and the peptide of Production Example 1 (10, 50, 100, 500µM), and a group treated with 5µg/ml of LPS and 10 µM of Dexamethasone (DEX) (positive control group) were treated with the above-described active substances and drugs, and then RNA was extracted after 3 hours to carry out RT-PCR. RNA was extracted from MRC-5 cells using an easy-BLUE^{™} Total RNA extraction kit (Qiagen, Germany). The extracted RNA was converted into cDNA using RT-PCR premix (iNtRON Biotechnology, Seongnam, Korea). After preparing a reaction mixture with PCR premix (iNtRON Biotechnology, Seongnam, Korea) and primers for each gene, PCR was carried out using a PCR machine (Eppendorf, Germany). Next, the mRNA expression pattern was determined by agarose gel electrophoresis. The base sequences of the primers used in the experiment are listed in Table 4 below.

**[Table 4]**

| Gene | Primer | Sequence (5' -> 3') | SEQ ID NO |
|---|---|---|---|
| α-SMA | F | GGTGCTGTCTCTCTATGCCTCTGGA | 14 |
| | R | CCCATCAGGCAACTCGTAACTCTTC | 15 |
| Fibronectin 1 | F | GGATGCTCCTGCTGTCAC | 18 |
| | R | CTGTTTGATCTGGACCTGCAG | 19 |
| Collagen 1A1 | F | CATCACCTACCACTGCAAGAAC | 16 |
| | R | ACGTCGAAGCCGAATTCC | 17 |
| Tenascin C | F | CCAGCGACCATCAACGCAGC | 20 |
| | R | GGGGCTTGTTCAGTGGATGCCT | 21 |
| GAPDH | F | GTG ATG GCA TGG ACT GTG GT | 12 |
| | R | GGA GCC AAA AGG GTC ATC AT | 13 |

As a result of the experiment, as shown in FIG. 7, although the expression of the α-SMA, Fibronectin 1, Collagen 1A1, and Tenascin C genes was increased in a case where cells were treated with TGF-β1, the treatment with the peptide of Production Example 1 decreased the expression of genes in a concentration-dependent manner.

### Experimental Example 5: Inhibition of inflammatory cytokine gene expression induced by IL-1β

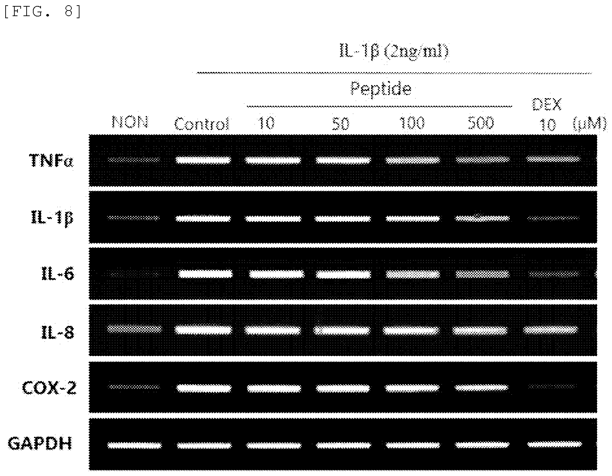

It was tested whether the peptide of SEQ ID NO: 1 produced in Production Example 1 inhibits the expression of inflammatory cytokine genes in lung cells. A549 cells (human alveolar basal epithelial cells) were seeded at 3 × 10⁵ cells/well in a 6-well plate and used in the experiment. After culturing for 1 day with a culture medium containing 10% FBS, the culture medium was replaced with a culture medium without containing FBS to induce starvation for 4 hours. A normal group without being treated with any substances, a group treated with IL-1β (2 ng/ml) (negative control group), groups treated with IL-1β (2 ng/ml) and the peptide of Production Example 1 (10, 50, 100, 500µM), and a group treated with IL-1β (2 ng/ml) and 10µM of Dexamethasone (DEX) (positive control group) were treated with the above-described active substances and drugs respectively, and then RNA was extracted after 3 hours to carry out RT-PCR. RNA was extracted from the A549 cells in each of the above groups using an easy-BLUE^{™} Total RNA extraction kit (Qiagen, Germany). The extracted RNA was converted into cDNA with RT-PCR premix (iNtRON Biotechnology, Seongnam, Korea). After preparing a reaction mixture with PCR premix (iNtRON Biotechnology, Seongnam, Korea) and primers for each gene, PCR was carried out using a PCR machine (Eppendorf, Germany). Next, the mRNA expression pattern was determined by agarose gel electrophoresis. The base sequences of the primers used in the experiment are as shown in [Table 2]. As a result of the experiment, as shown in FIG. 8, it was confirmed that although the treatment with IL-1β has increased the gene expression level of inflammatory cytokines TNF-α, IL-1β, IL-6, IL-8, and COX-2, the expression levels of the genes decreases in a concentration-dependent manner in a case where treatment is carried out with the peptide 1 of Production Example 1 is treated.

### Experimental Example 6: Inhibition of inflammatory cytokine protein expression induced by IL-1β

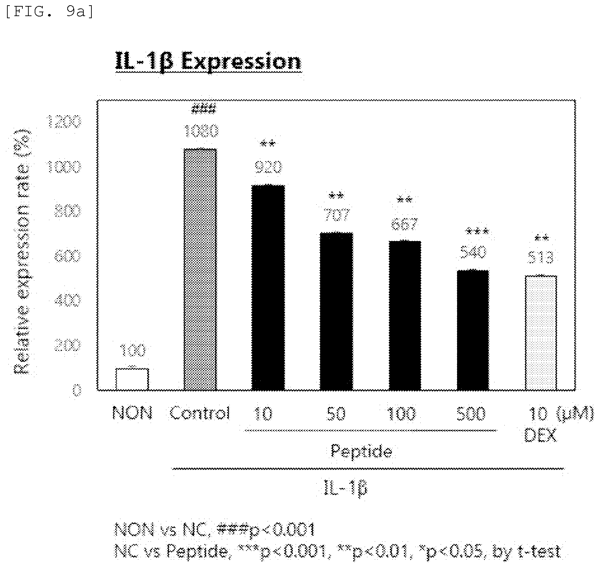

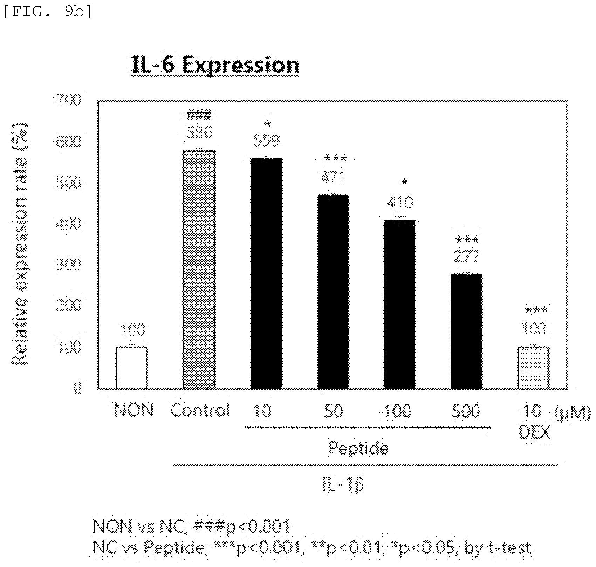

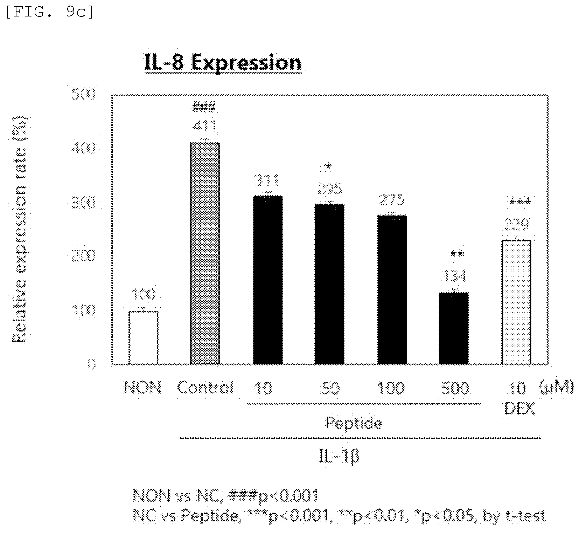

It was tested whether the peptide of SEQ ID NO: 1 produced in Production Example 1 inhibits the expression of the inflammatory cytokine proteins induced by IL-1β in lung cells. A549 cells (human alveolar basal epithelial cells) were seeded at 2 × 10⁵ cells/well in a 12-well plate and used in the experiment. After culturing for 1 day with a culture medium containing 10% FBS, the culture medium was replaced with a culture medium without containing FBS to induce starvation for 4 hours. Cells of a normal group (NON) without being treated with any substances, a group treated with IL-1β (2ng/ml) (negative control group, NC), groups treated with IL-1β (2ng/ml) and the peptide of Production Example 1 (10, 50, 100, 500µM), and a group treated with IL-1β (2ng/ml) and Dexamethasone (DEX) (10 µM) (positive control group) were treated with active substances and drugs respectively, and then cultured for 72 hours. After cell culture, the supernatant obtained by centrifuging the culture solution at 4°C and 3,000 rpm for 10 minutes was subjected to ELISA analysis using an IL-6 Quantikine ELISA Kit (R&D systems), an IL-8 Quantikine ELISA Kit (R&D systems), and an IL-1β Quantikine ELISA Kit (R&D systems) according to the method of use in the kit instructions, and then the absorbance was measured at 450 nm using a microplate reader. As a result of the experiment, as shown in FIG. 9a, FIG. 9b, and FIG. 9c, it was confirmed that although the expression levels of IL-1β, IL-6, and IL-8 have been increased in a case where treatment was carried out with IL-1β, the treatment with the peptide of Production Example 1 decreases the expression levels of IL-1β, IL-6, and IL-8 in a concentration-dependent manner.

### Experimental Example 7: Inhibition of acute lung inflammation induced by LPS

### 1. Animal experiment

BALB/c mice (Female/7W) were used in animal experiments, and an acute lung inflammation model was established by injection of LPS. As shown in [Table 5] below, 7-week-old BALB/c female mice were divided into 5 groups of 5 mice each, and then the experiment was carried out. On the 1st day, LPS (10 µg/50 µl/mouse) was administered intranasally to the G2 to G5 groups. 1 hour after LPS administration, the G3 and G4 groups were subjected to intranasal administration with the peptide of Production Example 1 (0.5 mg, 1 mg), and the G5 positive control group was subjected to intraperitoneal administration with Dexamethasone (100µg). 24 hours after the administration of the active substance and the drug, the mice were sacrificed to obtain the bronchoalveolar lavage fluid and the lung tissue, which were used in the next analysis.

**[Table 5]**

| Group | Sex | Number of animals and number | Test group | LPS | Peptide /Drug | Dose (mg/head) | Dose amount (mL/head) |
|---|---|---|---|---|---|---|---|
| G1 | Female | 5 (1-5) | Normal group | - | - | - | - |
| G2 | Female | 5 (6-10) | Negative control group | + | - | - | - |
| G3 | Female | 5 (11-15) | Experiment al group 1 | + | Peptide (IN) | 0.5mg | 0.05 mL |
| G4 | Female | 5 (16-20) | Experiment al group 2 | + | Peptide (IN) | 1mg | 0.05 mL |
| G5 | Female | 5 (21-25) | Positive control group | + | DEX (IP) | 100µg | 0.05 mL |

In [Table 5], Peptide : peptide of Production Example 1, DEX: Dexamethasone, IN: intranasal administration, IP: intraperitoneal administration.

### 2. Obtaining of bronchoalveolar lavage fluid (BALF) and counting of cells.

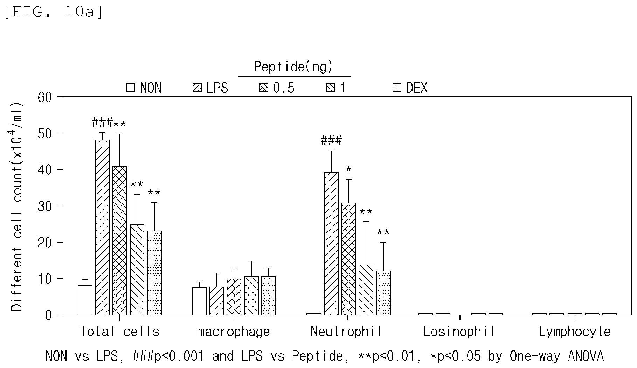

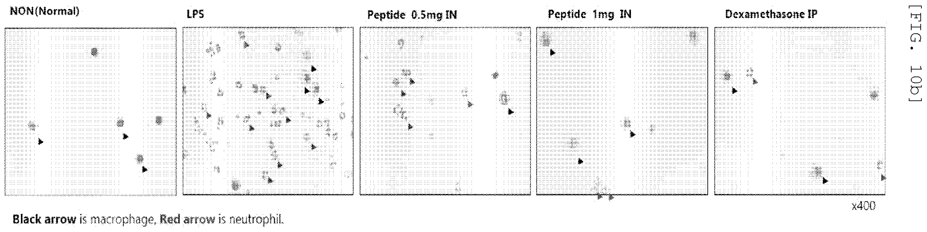

After anesthetizing the mouse, the treatment area was disinfected with 70% ethanol. Next, the skin was incised from the lower lip to the chest of the mouse, the airway was opened, and then the injection and recovery of 1ml of PBS were repeated twice through an IV catheter (BD, USA) to collect the bronchoalveolar lavage fluid (BALF). The collected BALF was centrifuged at 3,000 rpm for 10 minutes to separate the cells. The separated cells were resuspended in 0.5 ml of PBS, subsequently diluted with trypan blue solution at a 1:1 ratio, and then the total number of cells was counted using a hematocytometer. 10 µl of the remaining cell suspension was used to smear cells on a glass slide, and the cells were stained with a Diff-Quik staining solution (Sysmex, Japan) for a different cell count. After staining, cells were observed and counted through a microscope, and then the number of immune cells with respect to the total number of cells was calculated to show the results as a graph. As can be seen from the experimental results shown in FIG. 10a and FIG. 10b, the number of neutrophils was increased by an LPS treatment, and the treatment with the peptide of Production Example 1 significantly reduced the increased number of neutrophils in a concentration-dependent manner. The LPS treatment did not significantly increase the number of macrophages, which was not increased or decreased by the treatment with the peptide of Production Example 1.

### 3. Analysis of expression of pro-inflammatory proteins in bronchoalveolar lavage fluid (BALF)

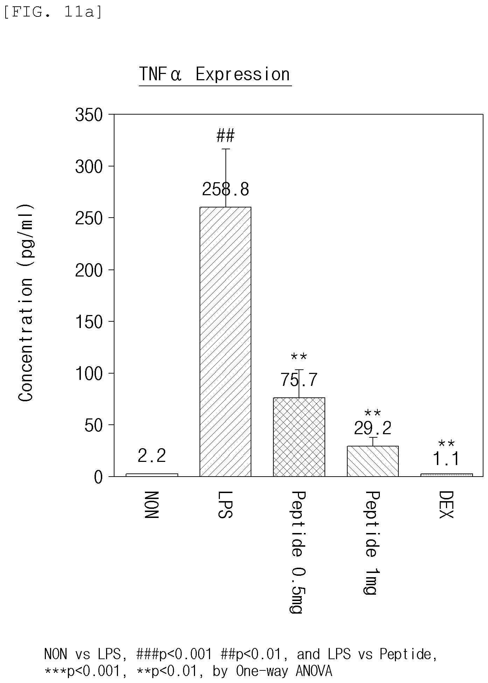

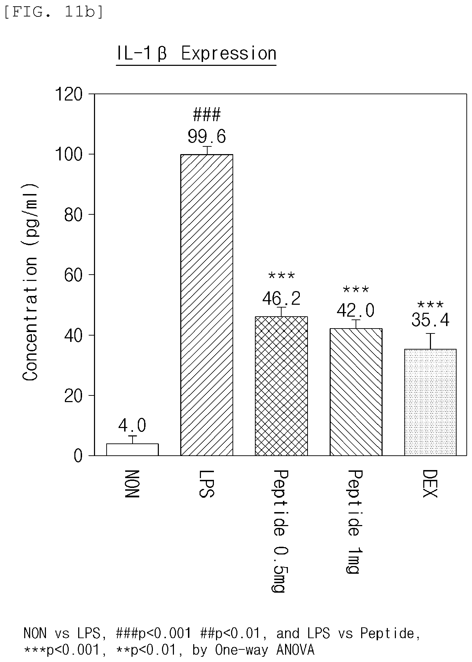

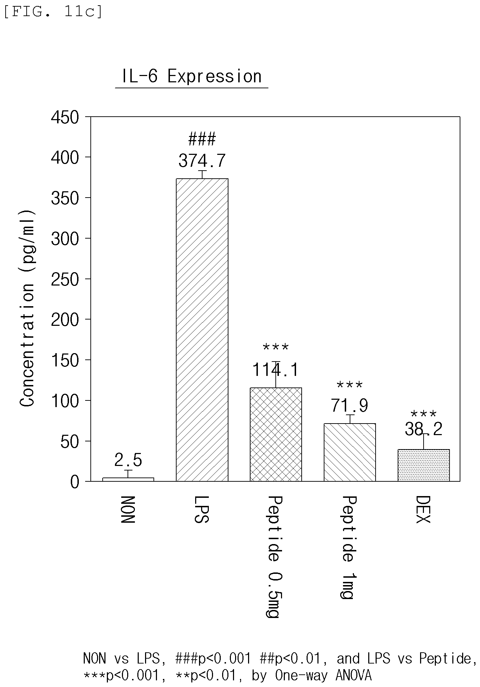

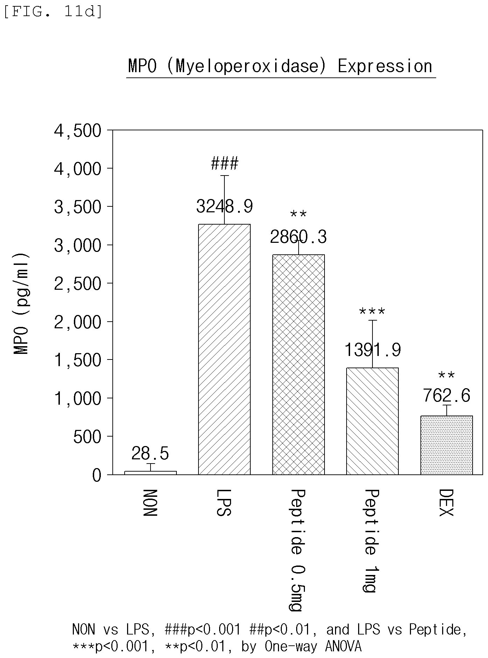

The collected BALF was subjected to the ELISA analysis of pro-inflammatory proteins. The supernatant obtained by centrifuging the collected BALF at 3,000 rpm for 10 minutes was subjected to analysis using a TNF-α Quantikine ELISA kit (R&D systems), an IL-1β Quantikine ELISA kit (R&D systems), an IL-6 Quantikine ELISA kit (R&D systems), and a Myeloperoxidase (MPO) Quantikine ELISA Kit (R&D systems) according to the instructions for use, and then the absorbance was measured at 450 nm using a microplate reader. As a result of the experiment, as can be seen in FIG. 11a to FIG. 11d, the expression levels of TNF-α, IL-1β, IL-6, and Myeloperoxidase (MPO) were increased by LPS administration, and the treatment with the peptide of Production Example 1 reduced the increased expression levels of the cytokines in a concentration-dependent manner.

### 4. Measurement of histological change in lung tissue

The lung tissue obtained from mice subjected to the animal experiment was washed with PBS at room temperature and then fixed with 4% paraformaldehyde (PBS dilution). The fixed tissue was washed three times with PBS and dehydrated using an ethanol series having a concentration gradient of 70% to 100%. Next, the lung tissue sample was embedded and cut into slices having a thickness of 4 µm. The sliced specimen having a size of 4 µm, which was placed on a slide glass, was dewaxed and hydrated in an ethanol series having a concentration gradient, and then stained sequentially in a hematoxlin solution for 1 minute and then in an Eosin solution for 10 seconds. After staining, the tissue slide was sequentially immersed in 90% and 100% ethanol, and finally, immersed in xylene twice for 5 minutes. Then, the tissue slide was mounted.

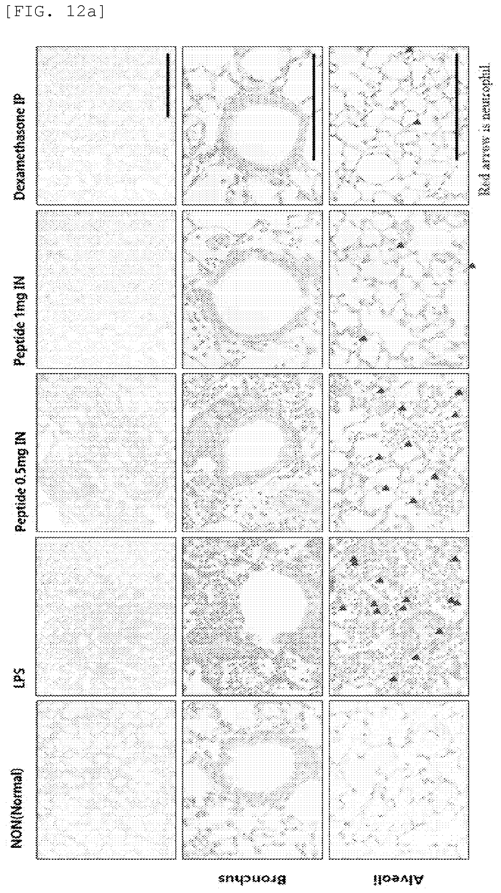

FIG. 12a shows the results obtained by observing the H&E staining of the lung tissue of mouse of the respective treatment groups.

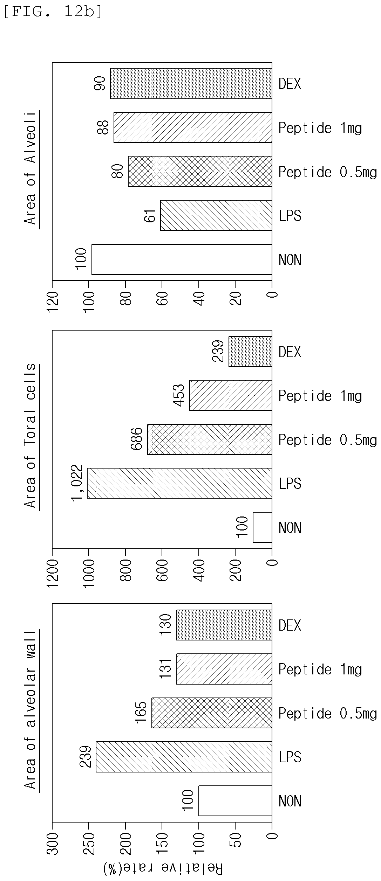

The graphs in FIG. 12b respectively show the results obtained by measuring the thickness of the alveolar walls (the alveolar wall thickness of the normal group is set to 100%, and the degree of increase in the thickness of the remaining treatment groups is indicated in terms of %), the results obtained by measuring the number of cells in the photographic image of the stained lung (the number of cells of the normal group is set to 100%, and the degree of increase in the number of cells of the remaining treatment groups is indicated in terms of %), and the results obtained by measuring the number of alveoli in the photographic image of the stained lung (the number of alveoli of the normal group is set to 100%, and the degree of increase or decrease in the number of alveoli of the remaining treatment groups is indicated in terms of %).

### Experimental Example 8: Inhibition of BLM-induced pulmonary fibrosis

### 1.Animal model of BLM-induced pulmonary fibrosis

As shown in [Table 6] below, 8-week-old BALB/c male mice were divided into 4 groups of 5 mice each. On the 1st day, a Bleomycin solution (10ng/50ul/mouse) was administered through the oropharynx to carry out one time of induction with BLM. Then, from the 7th day, the peptide of Production Example 1 (1 mg/mouse) was administered intranasally three times a week for 14 days, and Pirfeninone (2 mg/mouse) for a positive control group was administered orally three times a week for 14 days. On the 21st day of the induction with BLM, mice were sacrificed to obtain the bronchoalveolar lavage fluid and the lung tissue, which were used in the next analysis.

**[Table 6]**

| Group | Sex | Number of animals and number | Test group | BLM | Peptide /Drug | Dose (mg/head) | Dose amount (mL/head) |
|---|---|---|---|---|---|---|---|
| G1 | Male | 5 (1-5) | Normal group | - | - | - | - |
| G2 | Male | 5 (6-10) | Negative control group | + | - | - | - |
| G3 | Male | 5 (11-15) | Experiment al group | + | Peptide (IN) | 1 mg | 0.05 mL |
| G4 | Male | 5 (16-20) | Positive control group | + | Pirfeninone (Oral) | 2 mg | 0.1 mL |

In [Table 6], Peptide : peptide of Production Example 1, IN: intranasal administration, Oral: oral administration.

### 2. Body weight change

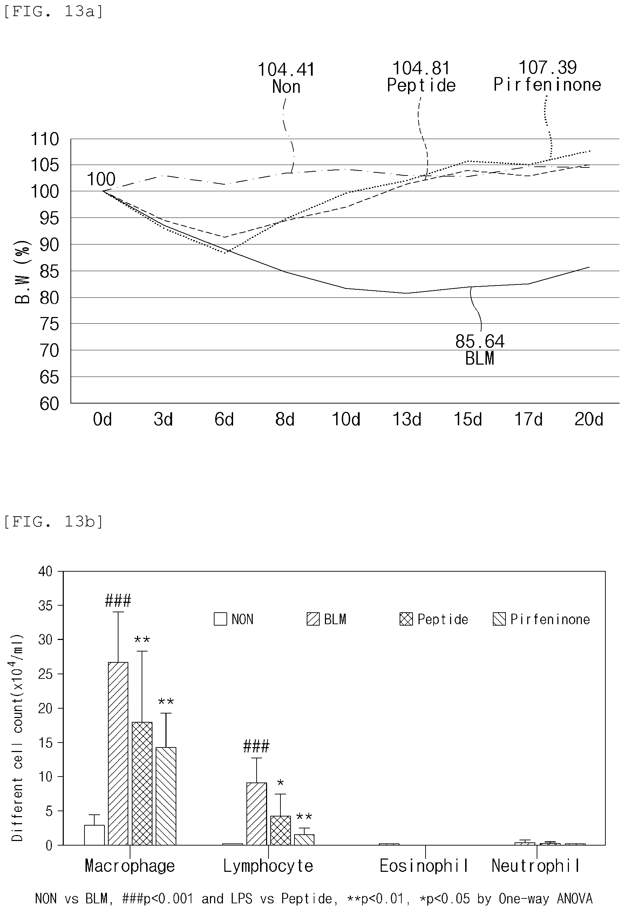

FIG. 13a shows the results obtained by measuring the body weight of the mice subjected to the animal experiment. The body weight was decreased in mice having BLM-induced pulmonary fibrosis, and the body weight, which had been decreased in a case where the peptide of Production Example 1 was administered, was recovered again.

### 3. Obtaining of bronchoalveolar lavage fluid (BALF) and counting of cells.

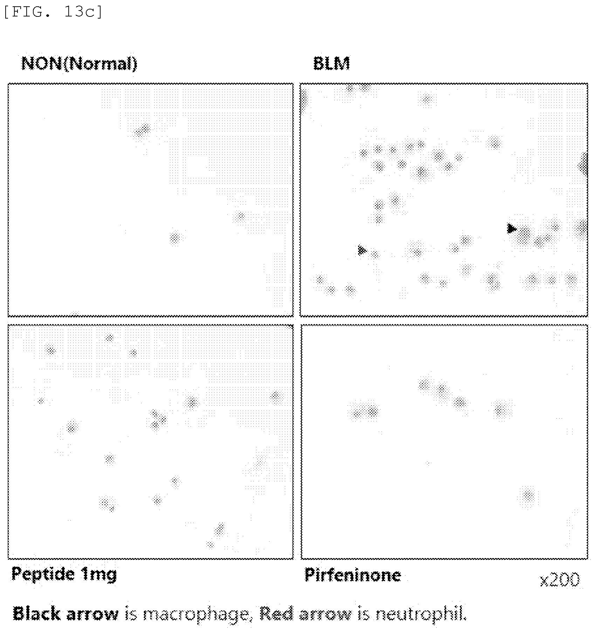

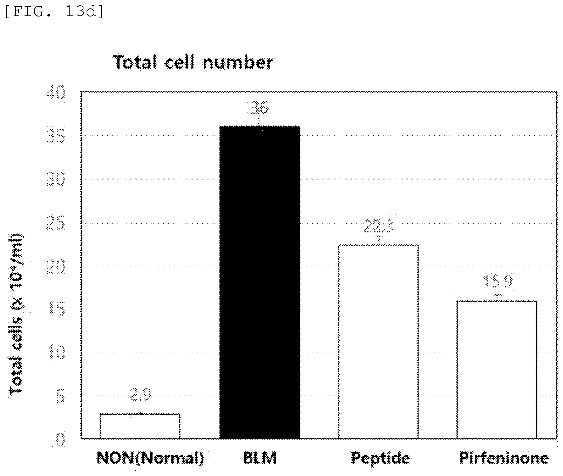

The mouse subjected to the animal experiment was anesthetized, and then the treatment area was disinfected with 70% ethanol. Next, the skin was incised from the lower lip to the chest of the mouse, the airway was opened, and then the injection and recovery of 1ml of PBS were repeated twice through an IV catheter (BD, USA) to collect the bronchoalveolar lavage fluid (BALF). The collected BALF was centrifuged at 3,000 rpm for 10 minutes to separate the cells. The separated cells were resuspended in 0.5 ml of PBS, subsequently diluted with trypan blue solution at a 1:1 ratio, and then the total number of cells was counted using a hematocytometer. 10 µl of the remaining cell suspension was used to smear cells on a glass slide, and the cells were stained with a Diff-Quik staining solution (Sysmex, Japan) for a different cell count. After staining, cells were observed and counted through a microscope, and then the number of immune cells with respect to the total number of cells was calculated to show the results as a graph. As can be seen from the experimental results shown in FIG. 13b, the number of macrophages and the number of lymphocytes were increased by a BLM treatment, and the treatment with the peptide of Production Example 1 decreased the increased number of macrophages and the increased number of lymphocytes. FIG. 13c and FIG. 13d show the results obtained by observing cells with a microscope and the results obtained by measuring the total number of cells.

### 4. Measurement of histological change in lung tissue - H&E staining

The lung tissue obtained from mice subjected to the animal experiment was washed with PBS at room temperature and then fixed with 4% paraformaldehyde (PBS dilution). The fixed tissue was washed three times with PBS and dehydrated using an ethanol series having a concentration gradient of 70% to 100%. Next, the lung tissue sample was embedded and cut into slices having a thickness of 4 µm. The sliced specimen having a size of 4 µm, which was placed on a slide glass, was dewaxed and hydrated in an ethanol series having a concentration gradient, and then stained sequentially in a hematoxlin solution for 1 minute and in an Eosin solution for 10 seconds. After staining, the tissue slide was sequentially immersed in 90% and 100% ethanol, and finally, immersed in xylene twice for 5 minutes. Then, the tissue slide was mounted.

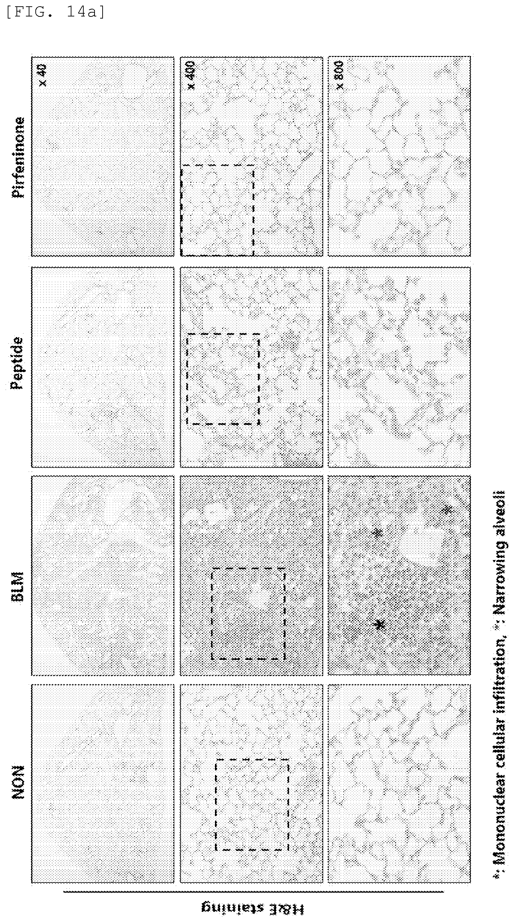

FIG. 14a shows the results obtained by observing the H&E staining of the lung tissue of mice of each treatment group.

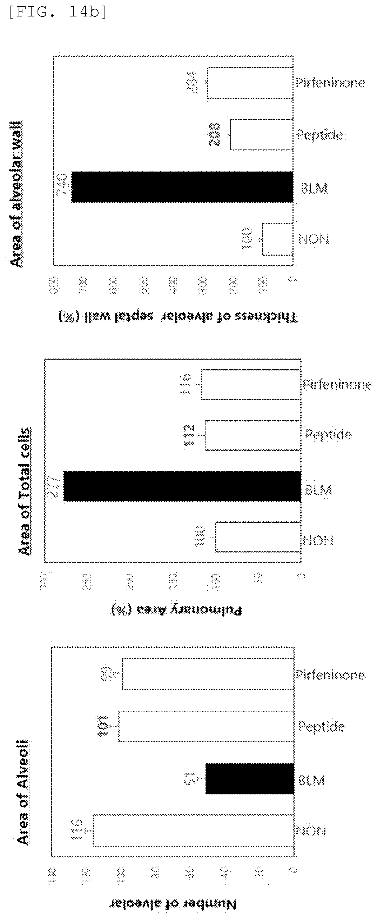

The graphs in FIG. 14b respectively show the results obtained by measuring the number of alveoli in each treatment group in an image of the lung tissue that has been subjected to the staining, the results obtained by measuring the total number of cells in the photographic image of the stained lung tissue (the total number of cells of the normal group is set to 100%, and the degree of increase in the total number of cells of the remaining treatment groups is indicated in terms of %), and the results obtained by measuring the thickness of the alveolar wall in each treatment group in an image of the lung tissue that has been subjected to the staining (the alveolar wall thickness of the normal group is set to 100%, and the degree of increase in the thickness of the remaining treatment groups is indicated in terms of %).

### 5. Measurement of histological change in lung tissue - Masson's Trichrome staining

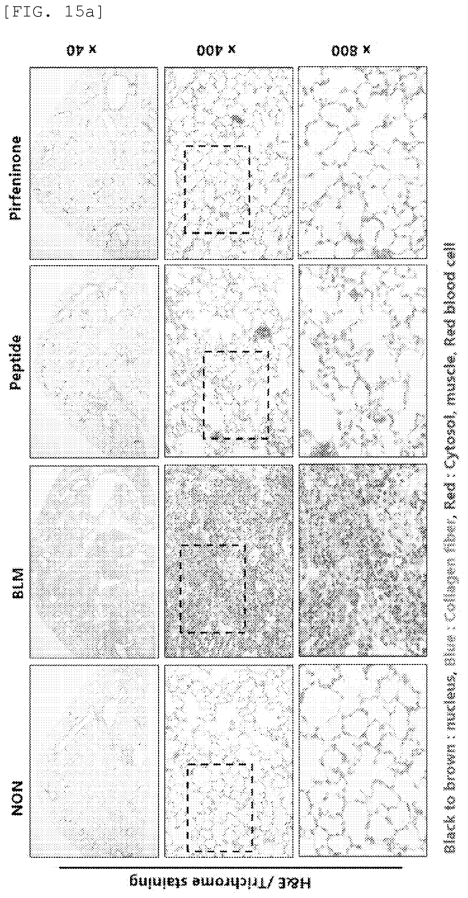

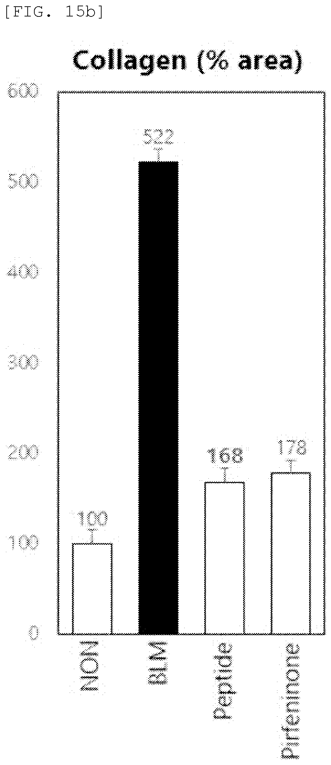

The lung tissue obtained from mice subjected to the animal experiment was washed with PBS at room temperature and then fixed with 4% paraformaldehyde (PBS dilution). The fixed tissue was washed three times with PBS and dehydrated using an ethanol series having a concentration gradient of 70% to 100%. Next, the lung tissue sample was embedded and cut into slices having a thickness of 4 µm. The sliced specimen having a size of 4 µm, which was placed on a slide glass, was dewaxed and hydrated in an ethanol series having a concentration gradient. Staining was carried out for 10 minutes in each of Weigert's iron hematoxlin, a Biebrich scarlet-acid fuchsin solution, a Phosphomolybdic-phosphotungstic acid solution, and an aniline blue solution. After completion of staining, the tissue slide was immersed in 1% acetic acid solution for 2 minutes and then washed with D.W. The tissue slide was sequentially immersed in 95% and 100% ethanol, and finally, immersed in xylene twice for 5 minutes. Then, the tissue slide was mounted. FIG. 15a shows the results obtained by staining collagen using the Trichrome staining method. FIG. 15b shows the change (in terms of %) in the amount of collagen in the remaining treatment groups comparative to the normal group, where the amount of collagen in the normal group is set to 100%. As a result of checking the degree of pulmonary fibrosis induced by the BLM treatment through the change in the amount of collagen, it was confirmed that the degree of collagen deposition in the lung tissue is significantly reduced in a case where treatment is carried out with the peptide of Production Example 1.

### Production Example 2: Production of pharmaceutical compositions

### 2-1. Production of powder

Peptide according to the present invention: 2 g
Lactose: 1 g

The above ingredients were mixed and filled into an airtight cloth to produce a powder.

### 2-2. Production of tablet

Peptide according to the present invention: 100 mg
Corn starch: 100 mg
Lactose: 100 mg
Magnesium stearate: 2 mg

After mixing the above ingredients, the resultant mixture was tableted according to a conventional production method for a tablet, whereby a tablet was produced.

### 2-3. Production of capsule

Peptide according to the present invention: 100 mg
Corn starch: 100 mg
Lactose: 100 mg
Magnesium stearate: 2 mg

After mixing the above ingredients, the resultant mixture was filled into a gelatin capsule according to a conventional production method for a capsule, whereby a capsule was produced.

### 2-4. Production of pill

Peptide according to the present invention: 1 g
Lactose: 1.5 g
Glycerin: 1.5 g
Xylitol: 0.5 g

After mixing the above ingredients, the resultant mixture was subjected to production according to a conventional method so that the amount was 4g per pill.

### 2-5. Production of granule

Peptide according to the present invention: 150 mg
Soybean extract: 50 mg
Glucose: 200 mg
Starch: 600 mg

After mixing the above ingredients, 100 mg of 30% ethanol was added to the resultant mixture, and drying was carried out at 60°C to form a granule, which was subsequently filled into a cloth.

### Production Example 3: Production of functional food composition

### 3-1. Production of healthy food

Peptide according to the present invention: 500 µg
Vitamin mixture: appropriate amount
Vitamin A Acetate: 70 mg
Vitamin E: 1.0 mg
Vitamin D: 0.13 mg
Vitamin B2: 0.15mg
Vitamin B6: 0.5 mg
Vitamin B12: 0.2 mg
Vitamin C: 10 mg
Biotin: 10 mg
Nicotinic acid amide: 1.7 mg
Folic acid: 50 mg
Calcium pantothenate: 0.5 mg
Mineral mixture: appropriate amount
Ferrous sulfate: 1.75 mg
Zinc oxide: 0.82 mg
Magnesium carbonate: 25.3 mg
Potassium phosphate, monobasic: 15 mg
Potassium phosphate, dibasic: 55 mg
Potassium citrate: 90 mg
Calcium carbonate: 100 mg
Magnesium chloride: 24.8 mg

The above-described vitamin mixture and mineral mixture had a compositional ratio such that they had a desirable composition of a mixture of ingredients relatively suitable for healthy food according to a desirable embodiment. However, the mixing ratio may be modified to have any ratio, and thus the above ingredients are mixed according to a conventional production method for healthy food, to produce granules, which can be subsequently used to produce a healthy food composition according to a conventional method.

### 3-2. Production of healthy drink

Peptide according to the present invention: 500 µg
Citric acid: 1,000 mg
Oligosaccharide: 100 g
Plum concentrate: 2 g
Taurine: 1 g
Total volume: 900 ml (purified water is added for volume adjustment)

After mixing the above ingredients according to a conventional production method for a healthy drink, the resultant mixture was stirred and heated at 85°C for about 1 hour, and then the resultant solution was filtered, placed in a sterilized container, sealed, sterilized, and subsequently stored in the refrigerator. Then, it was used for the production of a healthy drink composition. Although the above compositional ratio was such that it had a desirable composition of a mixture of ingredients relatively suitable for a favorite beverage according to a desirable embodiment, the mixing ratio can be modified to have any ratio depending on regional or ethnic preferences of class with demand, country with demand, intended use, or the like.

As described above, the representative exemplary embodiments of this application have been described; however, the scope of this application is not limited to the specific embodiments described above, and thus those skilled in the art will understand that appropriate changes may be made within the scope stated in the claims of this application.

## Claims

1. A pharmaceutical composition for treatment or prevention of a lung inflammatory disease, the pharmaceutical composition comprising a peptide having an amino acid sequence of SEQ ID NO: 1 as an active ingredient.

2. The pharmaceutical composition for treatment or prevention of a lung inflammatory disease, according to claim 1, wherein the lung inflammatory disease is a disease selected from the group consisting of asthma, allergic asthma, bronchitis, chronic bronchitis, acute bronchitis, bronchiolitis, diffuse panbronchiolitis, bronchiectasis, pleurisy, alveolitis, vasculitis, pneumonia, hypersensitivity pneumonia, chronic obstructive pulmonary disease, pulmonary fibrosis, idiopathic pulmonary fibrosis, cystic fibrosis, acute respiratory distress syndrome, acute lung injury, lung injury due to smoke inhalation, and heat-induced lung injury.

3. The pharmaceutical composition for treatment or prevention of a lung inflammatory disease, according to claim 1, wherein the peptide inhibits an expression of an inflammatory cytokine gene, an inflammatory cytokine protein, or a protein involved in inflammatory cytokine signaling in lung cells in which inflammatory responses are induced.

4. The pharmaceutical composition for treatment or prevention of a lung inflammatory disease, according to claim 3, wherein the inflammatory cytokine gene is one or more selected from the group consisting of TNFα, IL-1β, IL-6, IL-8, and COX-2 genes.

5. The pharmaceutical composition for treatment or prevention of a lung inflammatory disease, according to claim 3, wherein the inflammatory cytokine protein or the protein involved in the inflammatory cytokine signaling is one or more selected from the group consisting of IL-1β, IL-6, COX2, IL-8, p-JNK, p-ERK, p-PI3K, p-AKT, p-ERK, and myeloperoxidase.

6. The pharmaceutical composition for treatment or prevention of a lung inflammatory disease, according to claim 1, wherein the peptide inhibits an expression of a fibrosis gene in lung cells in which an inflammatory response is induced.

7. The pharmaceutical composition for treatment or prevention of a lung inflammatory disease, according to claim 6, wherein the fibrosis gene is one or more selected from the group consisting of α-SMA, Collagen 1A1, Fibronectin 1, and Tenascin C genes.

8. The pharmaceutical composition for treatment or prevention of a lung inflammatory disease, according to claim 1, wherein the peptide reduces the number of neutrophils in a lung tissue in which inflammatory responses are induced.

9. A functional food composition for amelioration or prevention of lung inflammation, the functional food composition comprising a peptide having an amino acid sequence of SEQ ID NO: 1 as an active ingredient.

10. A functional food composition for amelioration or prevention of pulmonary fibrosis, the functional food composition comprising a peptide having an amino acid sequence of SEQ ID NO: 1 as an active ingredient.
